(19) Europäisches Patentamt
European Patent Office
Office européen des brevets

(11) Veröffentlichungsnummer: **0 449 026 A2**

# (12) EUROPÄISCHE PATENTANMELDUNG

(21) Anmeldenummer: **91103726.5**

(22) Anmeldetag: **12.03.91**

(51) Int. Cl.5: **C07D 491/04**, C07D 211/46, A61K 31/445, //(C07D491/04, 319:00,221:00)

(30) Priorität: **24.03.90 DE 4009561**

(43) Veröffentlichungstag der Anmeldung: **02.10.91 Patentblatt 91/40**

(84) Benannte Vertragsstaaten: **AT BE CH DE DK ES FR GB GR IT LI LU NL SE**

(71) Anmelder: **BAYER AG**
**W-5090 Leverkusen 1 Bayerwerk(DE)**

(72) Erfinder: **Roeben, Wolfgang, Dr.**
**Strässchen Siefen 30**
**W-5060 Bergisch Gladbach 2(DE)**
Erfinder: **Lockhoff, Oswald, Dr.**
**Morgengraben 14**
**W-5000 Köln 80(DE)**
Erfinder: **Rossen, Kai, Dr.**
**Heymannstrasse 26**
**W-5090 Leverkusen 1(DE)**
Erfinder: **Schüller, Matthias, Dr.**
**Gellertweg 11**
**W-5600 Wuppertal 1(DE)**
Erfinder: **Stoltefuss, Jürgen, Dipl.-Ing.**
**Parkstrasse 20**
**W-5657 Haan 2(DE)**
Erfinder: **Paessens, Arnold, Dr.**
**Stresemannstrasse 51**
**W-5657 Haan(DE)**

## (54) Neue Desoxynojirimycinderivate, Verfahren zu ihrer Herstellung und ihre Verwendung in Arzneimitteln.

(57) Die Erfindung betrifft neue Desoxynojirimycinderivate, Verfahren zu ihrer Herstellung und ihre Verwendung in Arzneimitteln, insbesondere in antiviralen Arzneimitteln.

EP 0 449 026 A2

Die Erfindung betrifft neue Desoxynojirimycinderivate, Verfahren zu ihrer Herstellung und ihre Verwendung in Arzneimitteln, insbesondere in antiviralen Arzneimitteln.

Es ist bekannt, daß Derivate von 1-Desoxynojirimycin und 1-Desoxy-mannonojirimycin Glukosidase-Inhibitoren sind und zur Behandlung von Kohlenhydratstoffwechselerkrankungen eingesetzt werden [vgl. EP 34 784; DE 28 48 117, EP 193 770]. Außerdem sind 1-O-Methyl-N-benzoyl-dl-nojirimycine aus den Publikationen Chem. Pharm. Bull, 23 (11), 2567 - 2572 und Chem. Pharm. Bull. 26 (11), 3364 - 3372 bekannt. Ferner wird in der EP-A 315 017 die Verwendung von 2-Hydroxymethylen-3,4,5-trihydroxypiperidinen als antivirale Mittel beschrieben.

Außerdem wurden am 9.5.1990 einige O-acylierte Derivate des 1,5-Dideoxy-1,5-imino-D-glucitols und N-Alkyl, Acyl und Arylderivate publiziert (vgl. EP 0 367 748 A2).

Es wurden nun neue Desoxynojirimycinderivate der allgemeinen Formel (I),

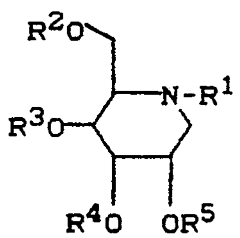

(I)

in welcher

$R^1$ für Wasserstoff oder
für geradkettiges oder verzweigtes Alkyl, Alkenyl oder Alkadienyl mit jeweils bis zu 10 Kohlenstoffatomen steht, die gegebenenfalls durch Halogen, geradkettiges oder verzweigtes Alkoxy mit bis zu 8 Kohlenstoffatomen oder durch Aryl oder Aryloxy mit 6 bis 10 Kohlenstoffatomen oder durch eine Gruppe der Formel $-CONR^6R^7$ substituiert sind,
worin

$R^6$ und $R^7$ gleich oder verschieden sind und Wasserstoff, geradkettiges oder verzweigtes Acyl oder Alkyl mit jeweils bis zu 8 Kohlenstoffatomen oder Phenyl bedeuten, das seinerseits durch geradkettiges oder verzweigtes Alkyl oder Alkoxy mit jeweils bis zu 8 Kohlenstoffatomen, Halogen oder Trifluormethyl substituiert sein kann.

$R^2$, $R^3$, $R^4$ und $R^5$ gleich oder verschieden sind und für Wasserstoff, oder
für eine Gruppe der Formel $-CO-R^8$ stehen,
worin

$R^8$

- geradkettiges oder verzweigtes Alkyl, Alkenyl oder Alkadienyl mit jeweils bis zu 30 Kohlenstoffatomen oder Aryl mit 6 bis 10 Kohlenstoffatomen bedeutet, das gegebenenfalls bis zu 2-fach gleich oder verschieden durch Halogen, Carboxy oder durch geradkettiges oder verzweigtes Alkyl, Alkoxy oder Alkoxycarbonyl mit jeweils bis zu 8 Kohlenstoffatomen substituiert ist, oder
- Cycloalkyl mit 3 bis 8 Kohlenstoffatomen bedeutet, das gegebenenfalls durch geradkettiges oder verzweigtes Alkyl mit bis zu 6 Kohlenstoffatomen substituiert ist, oder
- eine Gruppe der Formel $-NR^6R^7$ bedeutet,

worin

$R^6$ und $R^7$ die oben angegebene Bedeutung haben,

oder

$R^2$ und $R^3$, $R^3$ und $R^4$ oder R4 und $R^5$ gemeinsam für einen Rest der Formel

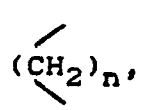

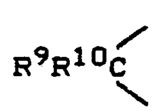

oder $O(SiR^{11}R^{12})_2$- stehen,
worin

n — die Zahl 1 oder 2 bedeutet,
und

$R^9$, $R^{10}$, $R^{11}$ und $R^{12}$ — gleich oder verschieden sind und geradkettiges oder verzweigtes Alkyl mit bis zu 8 Kohlenstoffatomen oder Phenyl bedeuten, das seinerseits durch geradkettiges oder verzweigtes Alkyl mit bis zu 8 Kohlenstoffatomen substituiert ist,

mit der Maßgabe, daß mindestens einer der oben angegebenen Substituenten $R^2$, $R^3$, $R^4$ oder $R^5$ nicht Wasserstoff bedeuten darf
und deren physiologisch unbedenklichen Salze.

Die erfindungsgemäßen Verbindungen der allgemeinen Formel (I) können in Form ihrer Salze vorliegen. Dies können Salze mit anorganischen oder organischen Säuren oder Basen sein. Zu den Säureadditionsprodukten gehören bevorzugt Salze mit Salzsäure, Bromwasserstoffsäure, Iodwasserstoffsäure, Schwefelsäure, Phosphorsäure oder mit Carbonsäuren wie Essigsäure, Propionsäure, Oxalsäure, Glykolsäure, Bernsteinsäure, Maleinsäure, Hydroxymaleinsäure, Methylmaleinsäure, Fumarsäure, Adipinsäure, Äpfelsäure, Weinsäure, Zitronensäure, Benzoesäure, Zimtsäure, Milchsäure, Ascorbinsäure, Salicylsäure, 2-Acetoxybenzoesäure, Nicotinsäure, Isonicotinsäure, oder Sulfonsäuren
wie Methansulfonsäure, Ethansulfonsäure, Benzolsulfonsäure, Toluolsulfonsäure, Naphthalin-2-sulfonsäure oder Naphthalindisulfonsäure.

Bevorzugt sind Verbindungen der allgemeinen Formel (I),
in welcher

$R^1$ — für Wasserstoff oder
- für geradkettiges oder verzweigtes Alkyl, Alkenyl oder Alkadienyl mit jeweils bis zu 8 Kohlenstoffatomen steht, die gegebenenfalls durch Fluor, Chlor, Brom, geradkettiges oder verzweigtes Alkoxy mit bis zu 8 Kohlenstoffatomen, Phenyl, Phenoxy oder durch eine Gruppe der Formel $-CONR^6R^7$ substituiert sind,

worin

$R^6$ und $R^7$ — gleich oder verschieden sind und
- Wasserstoff, geradkettiges oder verzweigtes Acyl oder Alkyl mit jeweils bis zu 6 Kohlenstoffatomen oder Phenyl bedeuten, das seinerseits durch geradkettiges oder verzweigtes Alkyl oder Alkoxy mit jeweils bis zu 6 Kohlenstoffatomen, Fluor, Chlor oder Trifluormethyl substituiert sein kann,

$R^2$, $R^3$, $R^4$ und $R^5$ — gleich oder verschieden sind und
- für Wasserstoff oder
- für eine Gruppe der Formel $-CO-R^8$ stehen,

worin

$R^8$ — geradkettiges oder verzweigtes Alkyl, Alkenyl oder Alkadienyl mit jeweils bis zu 25 Kohlenstoffatomen oder Phenyl bedeutet, das gegebenenfalls durch Fluor, Chlor, Brom, geradkettiges oder verzweigtes Alkyl mit bis zu 6 Kohlenstoffatomen substituiert sein kann, oder
- Cyclopropyl, Cyclopentyl oder Cyclohexyl bedeutet, das gegebenenfalls durch geradkettiges oder verzweigtes Alkyl mit bis zu 4 Kohlenstoffatomen substituiert ist,

oder

$R^8$ — eine Gruppe der Formel $-NR^6R^7$ bedeutet,
worin

$R^6$ und $R^7$ — die oben angegebene Bedeutung haben,

oder
$R^2$ und $R^3$ oder $R^3$ und $R^4$ oder $R^5$ und $R^6$ gemeinsam für einen Rest der Formel

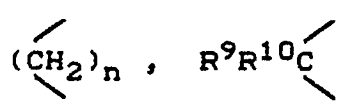

oder $O(SiR^{11}R^{12})_2$- stehen,

worin

n die Zahl 1 oder 2 bedeutet und

$R^9$, $R^{10}$, $R^{11}$ und $R^{12}$ gleich oder verschieden sind und geradkettiges oder verzweigtes Alkyl mit bis zu 6 Kohlenstoffatomen oder Phenyl bedeuten, das seinerseits durch geradkettiges oder verzweigtes Alkyl mit bis zu 6 Kohlenstoffatomen substituiert sein kann,

mit der Maßgabe, daß mindestens einer der Substituenten $R^2$, $R^3$, $R^4$ und $R^5$ nicht Wasserstoff bedeuten darf

und deren physiologisch unbedenklichen Salze.

Besonders bevorzugt sind Verbindungen der allgemeinen Formel (I),

in welcher

$R^1$ für Wasserstoff oder

- für geradkettiges oder verzweigtes Alkyl, Alkenyl oder Alkadienyl mit jeweils bis zu 6 Kohlenstoffatomen steht, die gegebenenfalls durch geradkettiges oder verzweigtes Alkoxy mit bis zu 6 Kohlenstoffatomen, Phenyl oder Phenoxy substituiert sind, oder durch eine Gruppe der Formel $-CONR^6R^7$ substituiert sind,

worin

$R^6$ und $R^7$ gleich oder verschieden sind und

- Wasserstoff, geradkettiges oder verzweigtes Alkyl mit bis zu 4 Kohlenstoffatomen oder Phenyl bedeuten, das seinerseits durch geradkettiges oder verzweigtes Alkyl mit bis zu 4 Kohlenstoffatomen substituiert sein kann,

$R^2$, $R^3$, $R^4$ und $R^5$ gleich oder verschieden sind und für Wasserstoff oder

- für eine Gruppe der Formel $-CO-R^8$ stehen,

worin

$R^8$ geradkettiges oder verzweigtes Alkyl, Alkenyl oder Alkadienyl mit jeweils bis zu 20 Kohlenstoffatomen oder Phenyl bedeutet, das gegebenenfalls durch geradkettiges oder verzweigtes Alkyl, Alkoxy oder Alkoxycarbonyl mit jeweils bis zu 4 Kohlenstoffatomen substituiert ist,

oder

$R^2$ und $R^3$ gemeinsam für den Rest der Formel

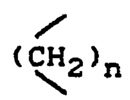 oder 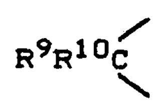

stehen,

worin

n - die Zahl 1 bedeutet,

$R^9$ und $R^{10}$ gleich oder verschieden sind und

- geradkettiges oder verzweigtes Alkyl mit bis zu 6 Kohlenstoffatomen oder Phenyl bedeuten, das seinerseits durch geradkettiges oder verzweigtes Alkyl mit bis zu 4 Kohlenstoffatomen substituiert ist,

mit der Maßgabe, daß mindestens einer der Substituenten $R^2$, $R^3$, $R^4$ und $R^5$ nicht Wasserstoff bedeuten darf

und deren physiologisch unbedenklichen Salze.

Außerdem wurden Verfahren zur Herstellung der Verbindungen der allgemeinen Formel (I) gefunden, dadurch gekennzeichnet, daß man

Verbindungen der allgemeinen Formel (II)

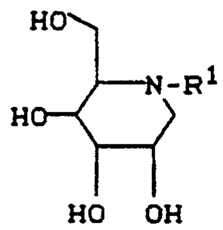

(II)

in welcher

$R^1$ die oben angegebene Bedeutung hat,

entweder

[A] direkt durch Umsetzung mit Verbindungen der allgemeinen Formeln (III), (IV) oder (V)

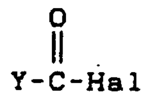 (III)

$O(CO\text{-}R^8)_2$ (IV)

oder $R^{13}\text{-}N=C=O$ (V)

in welchen

- Y den oben angegebenen Bedeutungsumfang von $R^2$, $R^3$, $R^4$ und $R^5$ umfaßt, aber nicht für Wasserstoff steht,
- Hal für Halogen, vorzugsweise für Chlor steht,
- $R^8$ die oben angegebene Bedeutung hat
- $R^{13}$ die oben angegebene Bedeutung von $R^6$ oder $R^7$ hat, aber nicht für Wasserstoff steht,

in inerten Lösemitteln acyliert oder carbamoyliert, oder

[B] zunächst durch Umsetzung mit Verbindungen der allgemeinen Formeln (VI) oder (VII)

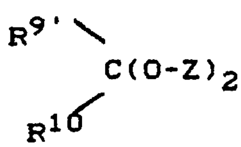 (VI)

oder $O(SiR^{11}R^{11}Hal)_2$ (VII)

in welcher

- $R^{9'}$ und $R^{10'}$ die oben angegebene Bedeutung von $R^9$ und $R^{10}$ haben aber nicht gleichzeitig für Wasserstoff stehen,
- Z für einen Alkylrest mit bis zu 6 Kohlenstoffatomen steht,
- $R^{11}$, $R^{12}$ und Hal die oben angegebene Bedeutung haben,

jeweils zwei Hydroxyschutzgruppen blockiert, anschließend nach der unter Verfahren [A] angegebenen Methoden selektiv acyliert oder carbamoyliert und in einem letzten Schritt nach üblicher Methode deblockiert.

Das erfindungsgemäße Verfahren kann durch folgendes Formelschema beispielhaft erläutert werden:

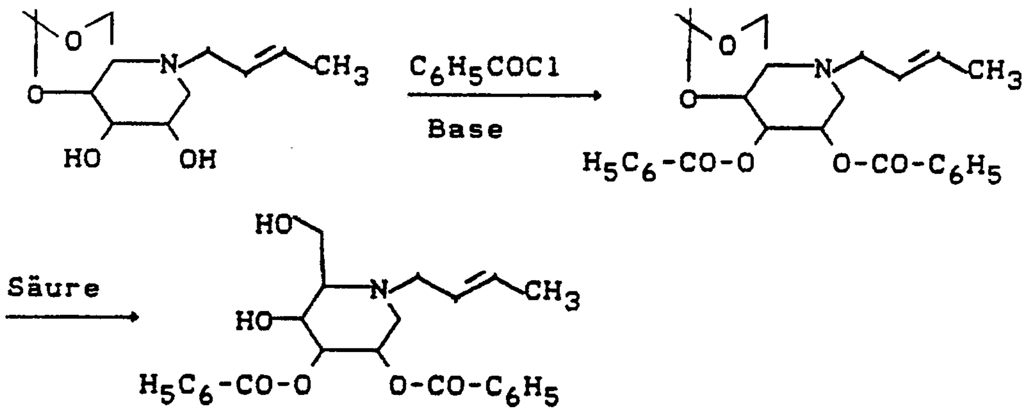


Als Lösemittel eignen sich die üblichen inerten Lösemittel, die sich unter den Reaktionsbedingungen nicht verändern. Hierzu gehören bevorzugt organische Lösemittel wie Kohlenwasserstoffe, beispielsweise Benzol, Toluol, Xylol, Cyclohexan, oder Halogenkohlenwasserstoffe wie Methylenchlorid, Chloroform, Tetrachlorkohlenwasserstoff oder Dimethylsulfoxid oder Dimethylformamid oder Ether wie Dioxan, Tetrahydrofuran oder Diethylether. Ebenso ist es möglich, Gemische der oben genannten Lösemittel einzusetzen. Bevorzugt sind Methylenchlorid, Chloroform und Dimethylformamid.

Als Basen eignen sich organische Amine (Trialkyl($C_1$-$C_6$)amine) wie beispielsweise Triethylamin oder Heterocyclen wie Pyridin, Methylpiperidin, Piperidin oder Morpholin. Bevorzugt ist Pyridin.

Die Base wird im allgemeinen in einer Menge von 0,5 bis 5 Mol, bevorzugt von 1 bis 3 Mol, bezogen auf 1 Mol der Verbindung der Formel (II) eingesetzt.

Die Acylierung, Acetalisierung und Carbamoylierung werden im allgemeinen in einem Temperaturbereich von 0° C bis +80° C, bevorzugt bei Raumtemperatur durchgeführt.

Als Acylierungsmittel eignen sich die üblichen literaturbekannten Chloride, wie beispielsweise Benzoylchlorid, Phenacetylchlorid oder n-Butyrylchlorid oder Anhydride wie beispielsweise Essigsäureanhydrid.

Die Blockierung (beispielsweise durch Acetalisierung) und Deblockierung der Hydroxyschutzgruppe erfolgt, gegebenenfalls säure- oder basenkatalysiert, nach literaturbekannten Methoden [vgl. Th. Greene: "Protective Groups in Organic Synthesis", J. Wiley and Sons, 1981, New York].

Als Carbamoylierungsmittel eignen sich beispielsweise Phenylisocyanat, p-Tolylisocyanat oder Methylisocyanat.

Die Verbindungen der allgemeinen Formel (II) sind an sich bekannt oder können nach üblicher Methode hergestellt werden [vgl. EP 7040].

Die Verbindungen der allgemeinen Formeln (III), (IV), (V), (VI) und (VII) sind bekannt oder können nach literaturbekannten Methoden hergestellt werden.

Es wurde überraschend gefunden, daß die Verbindungen der allgemeinen Formel (I) antiviral wirksam sind, insbesondere gegen Retroviren, das durch die weiter unten angegebenen Versuchsdaten beispielhaft belegt wird.

Das Visna-Virus und das HIV-Virus (Virus der humanen Immundefiziens) gehören beide zu der Retrovirus-Subfamilie der Lentiviren. Beide Viren weisen eine ähnliche Genomorganisation und ein gegenüber den anderen Retroviren komplexes Transkriptionsmuster auf [vgl. Sonijo, P et al., Cell (1985), 42, 369-382; Davis, J.L. et al., Journal of Virology (1987), 61 (5) 1325-1331].

Bekannte Inhibitoren des HIV hemmen auch das Visnavirus in vitro in vergleichbaren Konzentrationen d.h., dieses Modell eignet sich für die Prüfung und Auffindung von Hemmstoffen des HIV.

In Zellkulturen, die mit Visna-Virus infiziert sind, treten 5 bis 10 Tage nach der Infektion ausgeprägte virusinduzierte, zytopathische Effekte auf. Durch Behandlung der infizierten Zellkulturen mit den erfindungsgemäßen Verbindungen konnte das Auftreten dieser zytopathischen Effekte verhindert werden.

Der Visna-Virus-Test wurde nach der Methode von O. Narayan et al., Journal of Infectious Diseases 135, 5, 1977, 800 - 806 durchgeführt. Dazu wurden die erfindungsgemäßen Verbindungen im Kulturmedium in nicht zytotoxischen Konzentrationen in 96er Microtiterplatten verdünnt. Anschließend wurden Fibroblastenzellen vom Schaf (5 x $10^4$ Zellen pro Napf) in Produktionsmedium zu jedem Näpfchen zugeführt. Jedes Näpfchen erhielt dann 50 $\mu$l einer Visna-Viruslösung mit einer Titer von ca. 2,5 x $10^4$ $TCID_{50}$ (TCID = tissue culture infectious dosis). Diese Virusdosis entspricht einer MOI (Multiplizität der Infektion) von ca. 0,05.

Unter diesen Infektionsbedingungen resultierte zwischen Tag 5 und Tag 10 in einer Infektionskontrolle

ohne Substanz ein virusinduzierter, zytopathischer Effekt. Die infizierten und behandelten Zellen und die Kontrollzellen wurden 7 Tage bei 37° C 5% $CO_2$ inkubiert.

Bei Auftreten des virusinduzierten zytopathogenen Effektes in der unbehandelten Viruskontrolle wurden die Kulturen mit Formalin fixiert und anschließend mit einer Giemsa-Lösung gefärbt. Die inhibitorische Konzentration ($IC_{50}$) wurde mikroskopisch als die Konzentration ermittelt, bei der der zytopathische Effekt um 50% gehemmt wurde: im Vergleich zur unbehandelten Viruskontrolle, die 100% Zellzerstörung aufwies.

Es wurde gefunden, daß die erfindungsgemäßen Verbindungen mit Visna-Virus infizierten Zellen vor der virusinduzierten Zellzerstörung schützen.

**Tabelle**

| Beispiel-Nr. | $IC_{50}$ (µg/ml) |
|---|---|
| 3 | 50 |
| 4 | 20 |
| 6 | 12,5 |
| 10 | 20 |

Die erfindungsgemäßen Verbindungen stellen wertvolle Wirkstoffe zur Behandlung und Prophylaxe von Erkrankungen, hervorgerufen durch Retroviren, in der Human-und Tiermedizin dar.

Als Indikationsgebiete in der Humanmedizin können beispielsweise genannt werden:

1.) Die Behandlung oder Prophylaxe von menschlichen Retrovirusinfektionen.

2.) Für die Behandlung oder Prophylaxe von HIV 1 (Virus der humanen Immundefizienz; früher HTLV III/LAV genannt) und HIV II verursachten Erkrankungen (AIDS) und den damit assozierten Stadien wie ARC (AIDS related complex) und LAS (Lymphadenopathie-Syndrom) sowie der durch dieses Virus verursachten Immunschwäche und Encephalopathie.

3.) Für die Behandlung oder die Prophylaxe einer HTLV I-oder HTLV II-Infektion.

4.) Für die Behandlung oder die Prophylaxe des AIDS-carrier Zustandes (AIDS-Überträger-Zustand).

Als Indikationen in der Tiermedizin können beispielsweise angeführt werden:

Infektionen mit

a) Maedivisna (bei Schafen und Ziegen)

b) progressivem Pneumonievirus (PPV) (bei Schafen und Ziegen)

c) caprine arthritis encephalitis Virus (bei Schafen und Ziegen)

d) Zwoegerziekte Virus (bei Schafen)

e) infektiösem Virus der Anämie (des Pferdes)

f) Infektionen verursacht durch das Katzenleukämievirus

g) Infektionen verursacht durch das Virus der Katzen-Immundefizienz

Zur vorliegenden Erfindung gehören pharmazeutische Zubereitungen, die neben nicht-toxischen, inerten pharmazeutisch geeigneten Trägerstoffen eine oder mehrere Verbindungen der Formel (I) enthalten oder die aus einem oder mehreren Wirkstoffen der Formel (I) bestehen, sowie Verfahren zur Herstellung dieser Zubereitungen.

Die Wirkstoffe der Formel (I) sollen in den oben aufgeführten pharmazeutischen Zubereitungen, vorzugsweise in einer Konzentration von etwa 0,1 bis 99,5, vorzugsweise von etwa 0,5 bis 95 Gew.-%, der Gesamtmischung vorhanden sein.

Die oben aufgeführten pharmazeutischen Zubereitungen können außer den Verbindungen der Formel (I) auch weitere pharmazeutische Wirkstoffe enthalten.

Die Herstellung der oben aufgeführten pharmazeutischen Zubereitungen erfolgt in üblicher Weise nach bekannten Methoden, z-B- durch Mischen des oder der Wirkstoffe mit dem oder den Trägerstoffen.

Im allgemeinen hat es sich sowohl in der Human- als auch in der Veterinärmedizin als vorteilhaft erwiesen, den oder die Wirkstoffe der Formel (I) in Gesamtmengen von etwa 0,5 bis etwa 500, vorzugsweise 5 bis 100 mg/kg Körpergewicht je 24 Stunden, gegebenenfalls in Form mehrerer Einzelgaben, zur Erzielung der gewünschten Ergebnisse zu verabreichen. Eine Einzelgabe enthält den oder die Wirkstoffe

vorzugsweise in Mengen von etwa 1 bis etwa 80, insbesondere 1 bis 30 mg/kg Körpergewicht. Es kann jedoch erforderlich sein, von den genannten Dosierungen abzuweichen, und zwar in Abhängigkeit von der Art und dem Körpergewicht des zu behandelnden Objekts, der Art und der Schwere der Erkrankung, der Art der Zubereitung und der Applikation des Arzneimittels sowie dem Zeitraum bzw. Intervall, innerhalb welchem die Verabreichung erfolgt.

Herstellungbeispiele

Beispiel 1

N-Butenyl-4,6-isopropyliden-nojirimycin

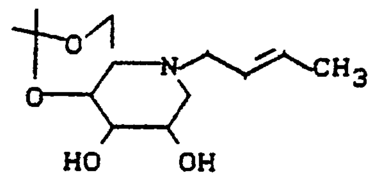

Eine Lösung von N-Butenylnojirimycin (22,8 g, 105 mmol) in 120 ml trockenem DMF wird mit wenigen Tropfen konzentrierter $H_2SO_4$ auf einen pH-Wert von 4 eingestellt. 160 ml 2,2-Dimethoxypropan werden zugegeben und die Lösung wird über Nacht bei 80 °C gerührt. Die Reaktionsmischung wird zu einem Überschuß von gesättigter $NaHCO_3$-Lösung gegeben, mit Methylenchlorid extrahiert und getrocknet. Flash-Chromatographie (Ethylacetat:MeOH = 9:1) ergibt 21,1 g eines hellgelben Öls (82 mmol, 78% Ausbeute)

Beispiel 2

N-Butenyl-2,3-dibenzoyl-4,6-isopropyliden-deoxyojirimycin

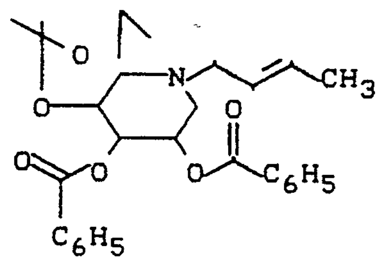

Zu einer Lösung von 500 mg N-Butenyl-4,6-isopropylidendesoxynojirimycin (1,9 mmol) in 3 ml trockenem Methylenchlorid werden 0,6 g Pyridin, 0,05 g 4-Dimethylaminopyridin und 0,8 g Benzoylchlorid gegeben. Die Reaktionsmischung wird bei Raumtemperatur über Nacht gerührt und dann durch Zugabe von 5 ml gesättigter $NaHCO_3$-Lösung gequenscht. Die Reaktionsmischung wird zu 150 ml tert.-Butylmethylether gegeben, mit gesättiger $NaHCO_3$-Lösung und gesättigter NaCl-Lösung gewaschen, über $MgSO_4$ getrocknet und eingedampft. Das Rohprodukt wird über $SiO_2$ chromatographiert (Ethylacetat:Hexan = 2:8) und ergibt 620 mg (1,3 mmol, 68% Ausbeute) Produkt als Öl.

Beispiel 3

N-Butenyl-2,3-dibenzoyl-desoxynojirimycin

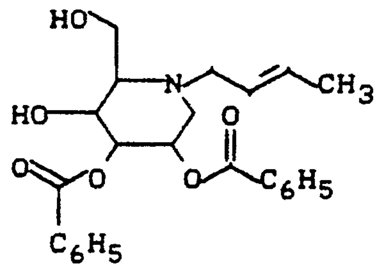

Zu einer Lösung von 620 mg der Verbindung aus Beispiel 2 (1,3 mmol) in 2 ml Tetrahydrofuran werden 1 ml 10%ige HCl gegeben. Die Reaktion wird über Nacht bei Raumtemperatur gerührt. Die Reaktion wird durch Zugabe von 5 ml gesättigter $NaHCO_3$-Lösung beendet und das Rohprodukt durch Extraktion mit Methylenchlorid gewonnen. Die Reinigung erfolgt durch Chromatographie auf einer präparativen DC-Platte (PSC Fertigplatte, Kieselgel 60, Merck AG) mit Ethylacetat:Hexan = 20:80). Es ergeben sich 166 mg der Titelverbindung (0,4 mmol, 30% Ausbeute) als weißer Feststoff mit einem Schmelzpunkt von 63-66° C.

Die in Tabelle 1 aufgeführten Beispiele wurden in Analogie zur Vorschrift der Beispiele 1, 2 und 3 hergestellt:

Tabelle 1

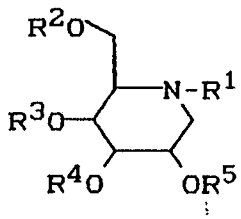

| Bsp.-Nr. | $R^1$ | $R^2$ | $R^3$ | $R^4$ | $R^5$ | $R_f$ |
|---|---|---|---|---|---|---|
| 4 | $-CH_2-CH=CH-CH_3$ | H | H | $-CO-C_9H_{19}$ | $-CO-C_9H_{19}$ | |
| 5 | H | H | H | $-CO-C_9H_{19}$ | $-CO-C_9H_{19}$ | |
| 6 | $-CH_2-CH=CH-CH_3$ | H | H | $-CO-CH_3$ | $-CO-CH_3$ | |
| 7 | H | $(CH_3)_2C$ | | $-CO-C_{13}H_{27}$ | $-CO-C_{13}H_{27}$ | |
| 8 | H | $(CH_3)_2C$ | | $-CO-C_9H_{19}$ | $-CO-C_9H_{19}$ | |
| 9 | $-(CH_2)_3-C_6H_5$ | $-CO-CH_3$ | $-COCH_3$ | $-CO-CH_3$ | $-CO-CH_3$ | |

**Fortsetzung Tabelle 1**

| Bsp.-Nr. | $R^1$ | $R^2$ | $R^3$ | $R^4$ | $R^5$ | $R_f$ |
|---|---|---|---|---|---|---|
| 10 | $-CH_2-CH=CH-CH_3$ | H | H | -CO-iButyl | -CO-iButyl | 0,59[a)] |
| 11 | $-CH_2-CH=CH-CH_3$ | H | H | $-CO-C_6H_5-mCH_3$ | $-CO-C_6H_5-mCH_3$ | 0,66[a)] |
| 12 | $-(CH_2)_5-O-C_6H_5$ | H | H | H | $-CO-(CH_2)_{14}-CH_3$ | |
| 13 | $-(CH_2)_5-O-C_6H_5$ | H | H | $-CO-(CH_2)_{14}-CH_3$ | H | |
| 14 | $-(CH_2)_5-O-C_6H_5$ | H | H | H | $-CO-(CH_2)_9-CH_3$ | |
| 15 | $-(CH_2)_5-O-C_6H_5$ | H | H | $-CO-(CH_2)_9-CH_3$ | H | |
| 16 | $-(CH_2)_5-O-C_6H_5$ | H | H | H | $-CO-(CH_2)_6-CH_3$ | |
| 17 | $-(CH_2)_5-O-C_6H_5$ | H | H | $-CO-(CH_2)_6-CH_3$ | H | |
| 18 | $-(CH_2)_5-O-C_6H_5$ | H | H | H | $-CO-(CH_2)_2-CH_3$ | |
| 19 | $-(CH_2)_5-O-C_6H_5$ | H | H | $-CO-(CH_2)_2-CH_3$ | H | |
| 20 | $-(CH_2)_5-O-C_6H_5$ | $-CO(CH_2)_2CH_3$ | $-CO(CH_2)_2CH_3$ | $-CO(CH_2)_2CH_3$ | H | |
| 21 | $-(CH_2)_5-O-C_6H_5$ | H | H | $-CO-(CH_2)_2-CH_3$ | $-CO(CH_2)_2CH_3$ | |

**Laufmittelsysteme:**

a) Essigester : Hexan = 1:1

Fortsetzung Tabelle 1

| Bsp.-Nr. | $R^1$ | $R^2$ | $R^3$ | $R^4$ | $R^5$ | $R_f$ |
|---|---|---|---|---|---|---|
| 22 | $-(CH_2)_2-\underset{OCH_3}{\underset{\vert}{CH}}-CH_3$ | H | H | H | H | |
| 23 | $-(CH_2)_2-\underset{OCH_3}{\underset{\vert}{CH}}-CH_3$ | $(CH_3)_2CH-$ | H | H | H | |
| 24 | $-(CH_2)_2-\underset{OCH_3}{\underset{\vert}{CH}}-CH_3$ | H | H | $-CO-CH_3$ | $-CO-CH_3$ | |
| 25 | $-(CH_2)_2-\underset{OCH_3}{\underset{\vert}{CH}}-CH_3$ | $-CO-C_3H_7$ | $-CO-C_3H_7$ | $-CO-C_3H_7$ | $-CO-C_3H_7$ | |
| 26 | $-CH_2-CH=CH-CH_3$ | H | H | H | $-CO-C_3H_7$ | |
| 27 | $-CH_2-CH=CH-CH_3$ | H | H | $-CO-C_3H_7$ | H | |

## Patentansprüche

1. Desoxynojirimycinderivate der allgemeinen Formel (I),

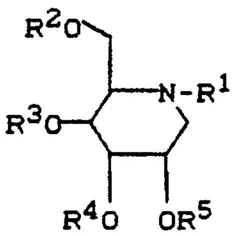

(I)

in welcher

$R^1$ für Wasserstoff oder für geradkettiges oder verzweigtes Alkyl, Alkenyl oder Alkadienyl mit jeweils bis zu 10 Kohlenstoffatomen steht, die gegebenenfalls durch Halogen, geradkettiges oder verzweigtes Alkoxy mit bis zu 8 Kohlenstoffatomen oder durch Aryl oder Aryloxy mit 6 bis 10 Kohlenstoffatomen oder durch eine Gruppe der Formel $-CONR^6R^7$ substituiert sind,

worin

$R^6$ und $R^7$ gleich oder verschieden sind und Wasserstoff, geradkettiges oder verzweigtes Acyl oder Alkyl mit jeweils bis zu 8 Kohlenstoffatomen oder Phenyl bedeuten, das seinerseits durch geradkettiges oder verzweigtes Alkyl oder Alkoxy mit jeweils bis zu 8 Kohlenstoffatomen, Halogen oder Trifluormethyl substituiert sein kann.

$R^2$, $R^3$, $R^4$ und $R^5$ gleich oder verschieden sind und für Wasserstoff, oder

für eine Gruppe der Formel $-CO-R^8$ stehen,

worin

$R^8$

- geradkettiges oder verzweigtes Alkyl, Alkenyl oder Alkadienyl mit jeweils bis zu 30 Kohlenstoffatomen oder Aryl mit 6 bis 10 Kohlenstoffatomen bedeutet, das gegebenenfalls bis zu 2-fach gleich oder verschieden durch Halogen, Carboxy oder durch geradkettiges oder verzweigtes Alkyl, Alkoxy oder Alkoxycarbonyl mit jeweils bis zu 8 Kohlenstoffatomen substituiert ist, oder
- Cycloalkyl mit 3 bis 8 Kohlenstoffatomen bedeutet, das gegebenenfalls durch geradkettiges oder verzweigtes Alkyl mit bis zu 6 Kohlenstoffatomen substituiert ist, oder
- eine Gruppe der Formel $-NR^6R^7$ bedeutet,

worin

$R^6$ und $R^7$ die oben angegebene Bedeutung haben,

oder

$R^2$ und $R^3$, $R^3$ und $R^4$ oder $R^4$ und $R^5$ gemeinsam für einen Rest der Formel

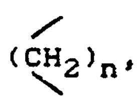, 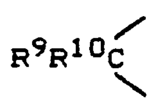

oder $O(SiR^{11}R^{12})_2-$ stehen,

worin

n die Zahl 1 oder 2 bedeutet,

und

$R^9$, $R^{10}$, $R^{11}$ und $R^{12}$ gleich oder verschieden sind und geradkettiges oder verzweigtes Alkyl mit bis zu 8 Kohlenstoffatomen oder Phenyl bedeuten, das seinerseits durch geradkettiges oder verzweigtes Alkyl mit bis zu 8 Kohlenstoffatomen substituiert ist,

mit der Maßgabe, daß mindestens einer der oben angegebenen Substituenten $R^2$, $R^3$, $R^4$ oder $R^5$ nicht Wasserstoff bedeuten darf

und deren physiologisch unbedenklichen Salze.

2. Verbindungen der allgemeinen Formel (I), nach Anspruch 1,

in welcher

$R^1$ für Wasserstoff oder
- für geradkettiges oder verzweigtes Alkyl, Alkenyl oder Alkadienyl mit jeweils bis zu 8 Kohlenstoffatomen steht, die gegebenenfalls durch Fluor, Chlor, Brom, geradkettiges oder verzweigtes Alkoxy mit bis zu 8 Kohlenstoffatomen, Phenyl, Phenoxy oder durch eine Gruppe der Formel $-CONR^6R^7$ substituiert sind, worin

$R^6$ und $R^7$ gleich oder verschieden sind und
- Wasserstoff, geradkettiges oder verzweigtes Acyl oder Alkyl mit jeweils bis zu 6 Kohlenstoffatomen oder Phenyl bedeuten, das seinerseits durch geradkettiges oder verzweigtes Alkyl oder Alkoxy mit jeweils bis zu 6 Kohlenstoffatomen, Flúor, Chlor oder Trifluormethyl substituiert sein kann,

$R^2$, $R^3$, $R^4$ und $R^5$ gleich oder verschieden sind und
- für Wasserstoff oder
- für eine Gruppe der Formel $-CO-R^8$ stehen,

worin

$R^8$ geradkettiges oder verzweigtes Alkyl, Alkenyl oder Alkadienyl mit jeweils bis zu 25 Kohlenstoffatomen oder Phenyl bedeutet, das gegebenenfalls durch Fluor, Chlor, Brom, geradkettiges oder verzweigtes Alkyl mit bis zu 6 Kohlenstoffatomen substituiert sein kann, oder
- Cyclopropyl, Cyclopentyl oder Cyclohexyl bedeutet, das gegebenenfalls durch geradkettiges oder verzweigtes Alkyl mit bis zu 4 Kohlenstoffatomen substituiert ist,

oder

$R^8$ eine Gruppe der Formel $-NR^6R^7$ bedeutet,

worin

$R^6$ und $R^7$ die oben angegebene Bedeutung haben,

oder

$R^2$ und $R^3$ oder $R^3$ und $R^4$ oder $R^5$ und $R^6$ gemeinsam für einen Rest der Formel

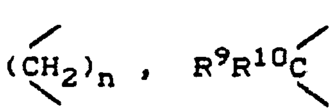

oder $O(SiR^{11}R^{12})_2$- stehen,

worin

n die Zahl 1 oder 2 bedeutet

und

$R^9$, $R^{10}$, $R^{11}$ und $R^{12}$ gleich oder verschieden sind und geradkettiges oder verzweigtes Alkyl mit bis zu 6 Kohlenstoffatomen oder Phenyl bedeuten, das seinerseits durch

geradkettiges oder verzweigtes Alkyl mit bis zu 6 Kohlenstoffatomen substituiert sein kann,

mit der Maßgabe, daß mindestens einer der Substituenten $R^2$, $R^3$, $R^4$ und $R^5$ nicht Wasserstoff bedeuten darf

und deren physiologisch unbedenklichen Salze.

**3.** Verbindungen der allgemeinen Formel (I) nach Anspruch 1,

in welcher

$R^1$ für Wasserstoff oder

- für geradkettiges oder verzweigtes Alkyl, Alkenyl oder Alkadienyl mit jeweils bis zu 6 Kohlenstoffatomen steht, die gegebenenfalls durch geradkettiges oder verzweigtes Alkoxy mit bis zu 6 Kohlenstoffatomen, Phenyl oder Phenoxy substituiert sind, oder durch eine Gruppe der Formel $-CONR^6R^7$ substituiert sind,

worin

R6 und $R^7$ gleich oder verschieden sind und

- Wasserstoff, geradkettiges oder verzweigtes Alkyl mit bis zu 4 Kohlenstoffatomen oder Phenyl bedeuten, das seinerseits durch geradkettiges oder verzweigtes Alkyl mit bis zu 4 Kohlenstoffatomen substituiert sein kann,

$R^2$, $R^3$, $R^4$ und $R^5$ gleich oder verschieden sind und für Wasserstoff oder

- für eine Gruppe der Formel $-CO-R^8$ stehen,

worin

$R^8$ geradkettiges oder verzweigtes Alkyl, Alkenyl oder Alkadienyl mit jeweils bis zu 20 Kohlenstoffatomen oder Phenyl bedeutet, das gegebenenfalls durch geradkettiges oder verzweigtes Alkyl, Alkoxy oder Alkoxycarbonyl mit jeweils bis zu 4 Kohlenstoffatomen substituiert ist,

oder

$R^2$ und $R^3$ gemeinsam für den Rest der Formel

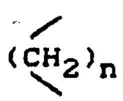 oder 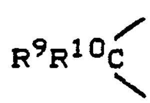

stehen,

worin

n - die Zahl 1 bedeutet,

$R^9$ und $R^{10}$ gleich oder verschieden sind und

- geradkettiges oder verzweigtes Alkyl mit bis zu 6 Kohlenstoffatomen oder Phenyl bedeuten, das seinerseits durch geradkettiges oder verzweigtes Alkyl mit bis zu 4 Kohlenstoffatomen substituiert ist,

mit der Maßgabe, daß mindestens einer der Substituenten $R^2$, $R^3$, $R^4$ und $R^5$ nicht Wasserstoff bedeuten darf

und deren physiologisch unbedenklichen Salze.

**4.** Verfahren zur Herstellung der Verbindungen gemäß den Ansprüchen 1 bis 3, dadurch gekennzeichnet, daß man

Verbindungen der allgemeinen Formel (II)

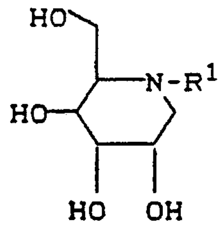

(II)

in welcher

$R^1$ die in den Ansprüchen 1 bis 3 angegebene Bedeutung hat,

entweder

[A] direkt durch Umsetzung mit Verbindungen der allgemeinen Formeln (III), (IV) oder (V)

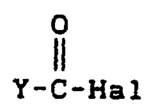

(III)

$O(CO\text{-}R^8)_2$ (IV)

oder $R^{13}\text{-}N=C=O$ (V)

in welchen

Y die in den Ansprüchen 1 bis 3 angegebenen Bedeutungsumfang von $R^2$, $R^3$, $R^4$ und $R^5$ umfaßt, aber nicht für Wasserstoff steht,

Hal für Halogen, vorzugsweise für Chlor steht,

$R^8$ die in den Ansprüchen 1 bis 3 angegebene Bedeutung hat

$R^{13}$ die in den Ansprüchen 1 bis 3 angegebene Bedeutung von $R^6$ oder $R^7$ hat, aber nicht für Wasserstoff steht,

in inerten Lösemitteln acyliert oder carbamoyliert, oder

[B] zunächst durch Umsetzung mit Verbindungen der allgemeinen Formeln (VI) oder (VII)

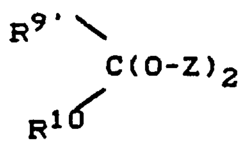

(VI)

oder $O(SiR^{11}R^{11}Hal)_2$ (VII)

in welcher

$R^{9'}$ und $R^{10'}$ die in den Ansprüchen 1 bis 3 angegebene Bedeutung von $R^9$ und $R^{10}$ haben aber nicht gleichzeitig für Wasserstoff stehen,

Z für einen Alkylrest mit bis zu 6 Kohlenstoffatomen steht,

$R^{11}$, $R^{12}$ und Hal die in den Ansprüchen 1 bis 3 angegebene Bedeutung haben,

jeweils zwei Hydroxyschutzgruppen blockiert, anschließend nach der unter Verfahren [A] angegebenen Methoden selektiv acyliert oder carbamoyliert und in einem letzten Schritt nach üblicher Methode deblockiert.

**5.** Arzneimittel enthaltend einen oder mehrere der Verbindungen gemäß den Ansprüchen 1 bis 3.